# DEMANDE DE BREVET EUROPEEN

(11) **EP 2 905 710 A1**
(43) Date de publication de la demande: **12.08.2015**
(21) Numéro de dépôt: 15154018.4
(22) Date de dépôt: 05.02.2015
(51) Int. Cl.: G06F 19/00, A61B 5/00, A61N 1/39, G06F 21/32

(54) **Dispositif de traitement de l'information doté de capteur(s) de donnée(s) physiologique(s)**

(30) Priorité: 06.02.2014 FR 1400340
(71) Demandeur: Ricard, Philippe, 06200 Nice (FR); Cadet, Pierre-Henri, 06400 Cannes (FR)
(72) Inventeur: Ricard, Philippe, 06200 Nice (FR); Cadet, Pierre-Henri, 06400 Cannes (FR)
(74) Mandataire: Schuffenecker, Thierry

(57) **Abrégé**

Un dispositif de traitement de l'information de type ordinateur, ordinateur portable, tablette tactile et/ou téléphone intelligent comportant :
- au moins un capteur pour la capture d'une donnée physiologique de l'utilisateur dudit dispositif de traitement de l'information;
- des moyens d'activation dudit capteur pour procéder à la capture en réponse à un événement périodique donné;
- des moyens de stockage pour le stockage de la donnée physiologique capturée en association avec une information de datage et/ou une information relative à l'utilisateur et/ou audit événement.

De préférence, l'événement correspondant au démarrage du système en sorte que lesdits moyens périodiques provoquent un stockage automatique d'une donnée physiologique datée à chaque démarrage de la machine en vue de la constitution d'une base de données physiologique propres à l'utilisateur.

## Description

### Domaine technique de l'invention

La présente invention concerne le milieu médical et notamment le domaine des dispositifs de traitement de l'information dotés de capteur(s) de donnée(s) physiologique (s) .

### Etat de la technique

Un suivi médical de qualité requiert une collecte de données médicales de précision et, si possible, se prolongeant toute la vie du patient.

De multiples systèmes sont aujourd'hui disponibles pour permettre la saisie de données physiologiques, telle que le pouls, la tension etc...

Néanmoins le développement de tous ces systèmes épars rend difficile une collecte de données susceptibles d'être rassemblées et stockée en vue de la constitution d'une base de données complète et qu'un utilisateur pourra exploiter toute sa vie durant.

Par ailleurs, se pose également la question de la sécurité des données saisies qui doivent être légitimement attribuées à leur titulaire véritable.

La présente invention vise à proposer à tout un chacun une solution élégante, efficace et performante permettant à un utilisateur de constituer une collection de données physiologiques d'un grand intérêt pour le cours de sa vie.

### Exposé de l'invention

La présente invention a pour but de proposer un nouveau dispositif de collecte de données physiologiques qui peuvent présenter un grand intérêt, tant pour un utilisateur donné que pour les Autorités de Santé Publique, permettant d'accroître l'efficacité des politiques publiques de prévention.

C'est un autre but de la présente invention que de permettre également la constitution d'une base de données physiologiques, proprement datées et sécurisées, permettant de soutenir un suivi médical de grande qualité tout le long de la vie d'un patient donné.

Accessoirement, l'invention a finalement pour but d'offrir également d'autres fonctionnalités médicales, comme la présence d'un défibrillateur installé, de fabrication ou simplement communiquant, au sein d'un dispositif aussi courant que l'est un ordinateur portable, en vue d'accroître la prévention de l'accident cardio-respiratoire.

La présente invention atteint ces objections en réalisant un dispositif de traitement de l'information de type ordinateur, ordinateur portable, tablette tactile et/ou téléphone intelligent comportant :
- au moins un capteur pour la capture d'une donnée physiologique de l'utilisateur dudit dispositif de traitement de l'information;
- des moyens d'activation dudit capteur pour procéder à la capture en réponse à un événement périodique donné;
- des moyens de stockage pour le stockage de la donnée physiologique capturée en association avec une information de datage et/ou une information relative à l'utilisateur et/ou audit événement.

De préférence, l'événement provoquant la collecte de la ou des données physiologiques est le démarrage du système en sorte que lesdits moyens périodiques provoquent un stockage automatique d'une donnée physiologique datée à chaque démarrage de la machine en vue de la constitution d'une base de données physiologique propres à l'utilisateur.

Alternativement, l'événement permettant de provoquer la collecte de données physiologiques pourra résulter du lancement d'un programme informatique ou d'une routine ou librairie du système d'exploitation de manière à constituer la réponse physiologique de l'utilisateur lors de l'utilisation dudit programme ou de ladite routine.

Dans un mode de réalisation particulier, la ou les données physiologiques sont stockées sur un support amovible , par exemple de type clé USB, ou transférés sur un serveur distant.

Dans un mode de réalisation particulier, le dispositif comportant un capteur infrarouge est localisé à proximité immédiate d'un capteur d'empreinte digitale présent à des fins de sécurité, de manière à assurer conjointement :
- la saisie de la courbe de pression et de fréquence cardiaque en même temps que la prise d'empreinte digitale destinée à l'activation du système ;
- la signature des courbes de pression et de fréquence cardiaque par une information dérivée du capteur d'empreinte digitale de manière à associer irréversiblement les données saisies par le capteur infrarouge et l'empreinte digitale de l'utilisateur

Dans un mode de réalisation spécifique, la donnée physiologique est une image de l'iris de l'oeil capturé au moyen d'une caméra présente disponible dans le système.

Alternativement ou cumulativement, la donnée physiologique est une empreinte vocale enregistrée par un dispositif audio présent dans le système, lorsque l'utilisateur répète une donnée prédéterminée.

Dans un autre mode de réalisation particulier, la donnée physiologique est une mesure de la saturation en oxygène réalisée au moyen d'un capteur utilisant deux rayonnements respectivement rouge et infrarouge.

Dans un autre mode de réalisation, le dispositif comporte deux électrodes, respectivement destinées à un contact avec l'index gauche et l'index droit, de manière à permettre la capture d'un Electro Cardiogramme .

Optionnellement, la donnée physiologique pourra résulter d'une mesure effectuée sur un prélèvement de larmes et/ou de salives, en vue de produire un examen de mesure de glycémie.

De préférence, la ou les données physiologiques sont capturées, datées et associées à une données caractéristiques de l'utilisateur, telle que son empreinte digitale disponible grâce à un capteur d'empreinte digitale présent dans le dispositif, en vue de sécuriser la capture d'information physiologiques.

Optionnellement, le dispositif pourra également comporte un défibrillateur intégré, de fabrication.

### Description des dessins

D'autres caractéristiques, but et avantages de l'invention apparaîtront à la lecture de la description et des dessins ci-après, donnés uniquement à titre d'exemples non limitatifs. Sur les dessins annexés :
La figure 1a illustre un premier mode de réalisation d'un dispositif de traitement d'information dotée de capteurs de données physiologiques en vue d'une collecte systématisée de données physiologiques.
La figure 1b illustre un procédé de capture de données physiologiques lors du démarrage d'un système d'exploitation.
La figure 2 illustre un second mode de réalisation se présentant sous la forme d'un ordinateur portable adapté pour une collecte systématisée de données ECG
La figure 3 illustre un mode de réalisation particulier d'un procédé de diagnostique automatique mis en oeuvre dans le second mode de réalisation.
La figure 4 illustre un troisième mode de réalisation d'un dispositif coopérant et communiquant avec un défibrillateur.
La figure 5 illustre un quatrième mode de réalisation d'un défibrillateur adapté à la coopération avec un dispositif de traitement de données de type tablette tactile.
La figure 6 illustre un cinquième mode de réalisation d'un défibrillateur adapté à la coopération avec un ordinateur portable ou ultra-portable, sous la forme d'un Kit-batterie spécifique.
La figure 7 illustre un sixième mode de réalisation d'un défibrillateur adapté à la coopération avec un ordinateur portable ou ultra-portable, sous la forme d'un caddie spécifique destiné à remplacer le module de lecteur de CD-DVD.

### Description des modes de réalisation préférés

### I. Premier mode : Un dispositif de traitement systématisant la capture de données physiologiques

### A) Architecture du dispositif

L'on décrit à présent comment l'on peut, de manière quasiment automatique et très sécurisée, constituer une gigantesque collecte de données physiologiques propres à un utilisateur et qui pourra se révéler, tout au long de sa vie, d'un grand intérêt.

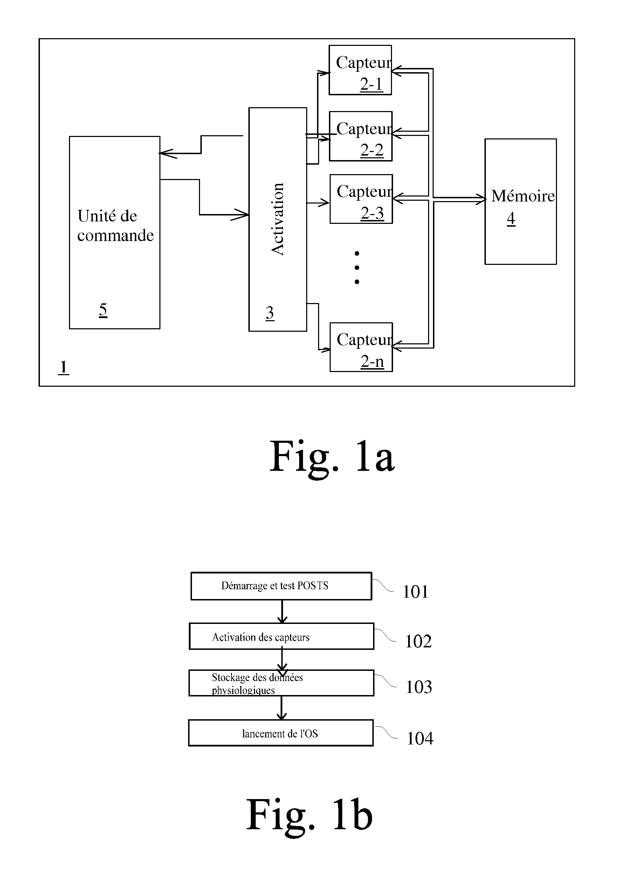

La figure 1 illustre plus particulièrement l'organisation centrale d'un dispositif de traitement de données 1 conforme à la présente invention et qui pourra se présenter sous la forme d'un ordinateur conventionnel, un ordinateur portable, une tablette tactile, voire même un téléphone dit intelligent. Dans un souci de ne pas alourdir l'exposé descriptif, les éléments que l'on retrouve conventionnellement dans un tel dispositif ne sont pas décrits plus avant.

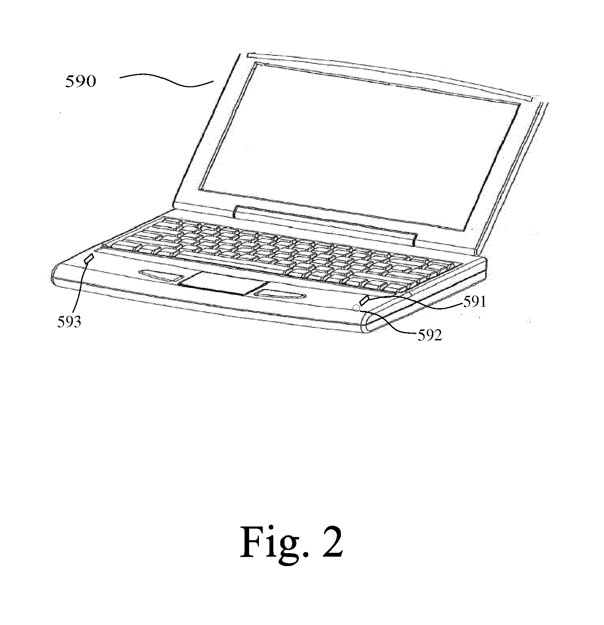

Le dispositif 1 comporte, en son sein, un ou plusieurs capteurs 2-1 à 2-n permettant la capture d'une ou plusieurs donnée(s) physiologique(s) de l'utilisateur du système. Divers capteurs pourront être envisagés. Par exemple, le capteur 2-1 pourra se composer d'un jeu d'électrodes, comme on le verra avec le dispositif de la figure 2, pour permettre un prélèvement d'ECG. Le capteur 2-2 pourra être tout simplement la caméra (webcam) disponible au sein du système. Le capteur 2-3 pourra être basé sur les fonctionnalités audio du dispositif, par exemple pour saisir une empreinte vocale. Le capteur 2-n pourra être constitué du capteur d'empreinte digitale conventionnel que l'on rencontre au sein d'un dispositif conventionnel.

De manière plus spécifique, le dispositif pourra comporter en outre divers capteurs spécifiques dédiés à la capteur de données physiologiques précises, comme par exemple des capteurs spécialisés de larmes ou de la salive pour permettre une mesure de glucose etc...

Dans un mode de réalisation particulier, l'on pourra également utiliser des périphériques adéquats communicant avec le dispositif via des moyens de communications dédiés, par exemple Bluetooth.

De préférence, l'on combine la capture de plusieurs données physiologiques, comme celles qui seront décrites ci-après dans la partie B.

Le dispositif 1 comporte en outre des moyens d'activation 3 des capteurs permettant de réaliser une activation systématique, à des instants judicieusement choisis, du ou des capteurs 2-1 à 2-n, en réponse à des événements prédéterminés.

Enfin, le dispositif 1 comporte des moyens de stockage 4 permettant le stockage de la ou des données physiologiques capturées en combinaison avec des informations de datage, ou relative à l'utilisateur voire même à un événement spécifique. Ces moyens de stockage pourront être réalisées au moyen de la mémoire conventionnelle du système, notamment au sein du disque dur, au d'une partition dédiée et protégée de celui-ci, voire même au sein d'une mémoire spécifique. Dans un mode de réalisation, l'on pourra utiliser une mémoire Flash ou une clé USB.

L'ensemble des circuits de capture, d'activation et de stockage des informations physiologiques est sous la commande d'une unité de commande 5 qui pourra être - dans un but d'économie de réalisation - soit le processeur principal de la machine, soit un circuit spécifiquement dédié à cette tâche qui pourra alors servir lors du démarrage du processeur principal ou lorsque ce dernier sera employé à d'autres tâches.

L'on constate dès à présent que la mise en oeuvre de l'invention ne nécessite que des modifications limitées d'un dispositif conventionnel de traitement de données, tel qu'un ordinateur portable ou une table tactile bien connue.

D'une manière générale, l'on utilisera avantageusement les possibilités de stockage et/ou de communication qui sont disponibles au sein du système (non représentés dans la figure 1).

Ainsi, les données physiologiques pourront également résulter d'une communication directe, notamment via un standard tel que Bluetooth, entre le dispositif et un périphérique adéquat permettant la capture de la donnée physiologique.

Plus simplement, les inventeurs ont découvert qu'il était possible de tirer avantage des fonctionnalités (audio, vidéo), déjà présentes au sein du système dans le but de permettre la collecte d'un grand nombre de données physiologiques et, à terme, la constitution d'une base de données d'un grand intérêt.

Cette liste de données physiologique va correspondre à autant d'informations qui seront stockées en vue de la constitution d'une base de données physiologiques qui sera destinée à servir:
- au propriétaire du système qui pourra ainsi, dès son plus jeune age et toute sa vie durant, constituer et développer une base de connaissances contenant des données d'un grand intérêt pour son suivi médical, et notamment en fin de vie lorsque le suivi apparaît plus critique.
- mais également de manière plus générale à la collectivité dans une perspective de Santé Publique qui pourra ainsi bénéficier de données statistiques d'un grand intérêt, tout en restant anonyme... Seul des profils extraits de la base de données pourront être transmis par exemple.

L'on notera en particulier le grand intérêt que présente l'invention de permettre la constitution d'une base de connaissance comportant des données physiologiques spécifiques au propriétaire du système, proprement datées et signées électroniquement, et qui pourront être ultérieurement traités par de multiples algorithmes et traitements qui resteront à définir.

L'invention permet ainsi la préparation d'une collecte de données d'une grande flexibilité puisqu'il n'est pas nécessaire, dans ce premier temps, de définir dès à présent tous les algorithmes qui seront utiles et opportuns pour une exploitation optimale des données.

Il importe simplement, ce que permet l'invention, d'organiser une collecte efficace, systématique de données proprement datées et sécurisées. D'une manière générale, la collecte de données physiologiques pourra être systématisée et résulter de multiples événements.

Dans un mode de réalisation illustré dans la **figure 1b****,** l'on voit que pour permettre la constitution de cette base de données, l'on pourra avantageusement prévoir des circuits de collecte actifs lors du démarrage de la machine, en sorte que l'on pourra ainsi mettre à profit le temps de démarrage du système pour assurer la capture et le stockage des données, et ce pendant que dure le démarrage du système d'exploitation de la machine. De préférence, le dispositif peut comporter des moyens pour différer le démarrage du système d'exploitation ou de certaines de ses fonctionnalités (routine logicielle, application), tant que la collecte des paramètres physiologiques n'a pas eu lieu. De cette manière, l'on réalise une sorte de « point de passage obligé » imposant à l'utilisateur une collecte rapide mais néanmoins systématique de données physiologiques qui viendront peu à peu enrichir la base de données lui étant attribuée.

Plus spécifiquement, le procédé démarre avec une étape 101 qui est le démarrage de la machine avec, en général les tests usuels matériels mis en oeuvre lors de la mise sous tension, dits POST (*Power On System Tests* en anglais)

Puis, dans une étape 102, l'unité de commande 5 - qui prendra dans cette configuration la forme d'un circuit spécifique dédié opérationnel bien avant le système d'exploitation - commande l'activation et la capture de données physiologiques - pendant la phase de démarrage du système d'exploitation, laquelle peut en général durer une dizaine de secondes.

Puis le procédé procède au stockage des données physiologiques au sein de la mémoire 4, lors d'une étape 103.

Enfin, le procédé s'achève par une étape 104 qui est le lancement définitif du système d'exploitation, en sorte que l'utilisateur peut alors utiliser son dispositif de traitement pour ses tâches quotidiennes, à savoir la lecture de ses courriels, la navigation sur Internet ou toute tâche quelconque.

Le mode de réalisation illustré dans la figure 1B présente l'avantage de systématiser, presque sans contrainte, la capture de données physiologiques pendant le temps nécessaire au démarrage du système, ce qui est un grand avantage de ce mode de réalisation.

Alternativement, le dispositif pourra comporter des moyens permettant l'activation automatique de la saisie lors d'un ou plusieurs événements spécifiques, comme par exemple le lancement ou le déroulement d'un programme ou d'un logiciel, la mise en oeuvre d'une routine ou d'une librairie logicielle (connue sous l'appellation anglaise *Dynamic Link Library -* DLL) etc... l'on pourra alors associer à la réponse physiologique des données relatives aux programmes (DLL, logiciels) en cours d'utilisation de manière à constituer une base de données d'un grand intérêt pour les éditeurs de logiciels qui vont pouvoir bénéficier ainsi de données concrètes utiles pour le développement, la correction ou l'évolution de leurs logiciels et notamment leur interface utilisateur (en anglais GUI - *Graphical User Interface*).

Pour prendre, par exemple, le cas du constructeur d'un système d'exploitation comme l'est MICROSOFT, ce dernier pourra alors effectuer un suivi permanent - et non anonyme - de l'utilisation de ses logiciels et notamment de son système d'exploitation WINDOWS et la manière dont celui-ci est « ressenti » par ses utilisateurs. Dans une telle perspective, l'on parvient ainsi à la mise en place d'un suivi "médical" à grande échelle de la "réponse physiologique" de l'utilisateur d'un ordinateur, non seulement en fonction de son age, mais également en fonction des tâches effectuées au moment de la collecte, des logiciels , routines, et même des pages web visualisées etc...

Toutes ces informations statistiques permettront un travail de corrélation d'un grand intérêt, notamment pour le suivi médical de l'utilisateur de l'ordinateur...

Mais il ne s'agit ici que d'une application spécifique.

Plus simplement, le dispositif décrit permet la collecte, de manière systématique, d'une ou plusieurs données physiologiques sur une longue période de temps en permettant notamment de dater correctement ces données physiologiques et, surtout, de les sécuriser.

A cet effet, les moyens de stockage des données physiologiques assurent le stockage des données combinées à une information propre et personnelle à l'utilisateur du système, et spécifiquement l'empreinte digitale saisie lors du démarrage du système.

Ainsi, l'on parvient à assurer que la base de données ne comporte que des données physiologiques afférentes à un même individu.

Dans un mode de réalisation particulier, les données physiologiques sont stockées sur un support amovible , par exemple de type clé USB, Ainsi, l'utilisateur d'un système pourra très simplement passer d'un système à un autre tout en conservant la possibilité d'enrichir aisément sa base de données spécifiques.

Alternativement, les données pourront être transmises vers un serveur sécurisé distant grâce aux moyens de communication disponible dans le dispositif de manière à permettre la constitution d'une base de données gérée sur un serveur distant.

### B) Données physiologiques collectées

Les éléments ci-dessous ne sont que des exemples non limitatifs de réalisation.

### BIOMETRIE au sens large : capture d'images par la caméra intégrée (webcam)

Le dispositif comporte une caméra 6 utilisée pour une capture d'une image de l'iris de l'oeil voir d'autres éléments tels que le « *gérontoxon* » (sorte de cercle sur l'iris), lequel s'avère être un indicateur pertinent de la cholestérolémie.

L'image capturée de l'iris et des éléments associés peut ainsi être stockée dans les moyens de stockage et à des fins d'analyse et/ou d'archivage .

De telles images peuvent se révéler être d'un grand intérêt pour le patient/utilisateur du système. En effet, la dilatation de la pupille ou mydriase, est un indicateur de possibles intoxications par les drogues, par certains médicaments, des poisons, des lésions cérébrales graves type traumatisme crâniens, anévrisme cérébral, oedème cérébral, hémorragie etc

La caméra 6 peut plus généralement utilisée pour la capture d'une image de la morphologie du visage en vue d'une comparaison de son évolution dans le temps. Une approche également intéressante en morpho psychologie pour l'analyse de la personnalité par exemple.

On parle aussi d'analyse comportementale

### Capture Audio : L'empreinte vocale

L'on utilise à présent avantageusement les fonctionnalités audio disponibles au sein du système en vue, notamment, d'une analysie de la cohérence d'un texte par rapport à une donnée enregistrée et considérée comme référence, basée sur l'analyse spectrale de la voix (harmoniques, modélisation de phonèmes) etc.

Il est intéressant en effet de noter que l'altération d'une ou plusieurs composantes peut être révélatrice de pathologies physiologiques ou psychologiques.

### Oxymétrie

Le dispositif est équipé à cette fin de capteurs de contact, pouvant notamment servir pour la capture du pouls et/ou d'un enregistrement ECG.

Sur ce même capteur de contact, l'on peut disposer un capteur supplémentaire émettant deux rayonnement spécifiquement rouge et infra-rouge dans le but de mesurer la saturation en oxygène. Parmi les fonctions diagnostiques simples il est possible de surveiller la fonction respiratoire, le pouls et la perfusion périphérique etc.

### La mesure de l'ECG

Le disposition 1 comportant deux capteur digitaux adéquats (index, index) permettant une analyse rapide d'un échantillon d'électrocardiogramme, sa mémorisation par rapport à un signal dit « normal », la mesure de la variabilité de la fréquence. Cette mesure peut être un indicateur d'une « arythmie silencieuse » révélatrice d'un trouble du rythme inconnu.
Le rajout d'une électrode autocollante ou la mise en place d'un petit boîtier Bluetooth au niveau du sternum permettrait la surveillance de la température, de la respiration, de l'apnée du sommeil par exemple.

### La mesure de la Glycémie

Dans un mode de réalisation particulier, l'on peut procéder à une analyse glucose oxydase au moyen d'un système non invasif. En particulier, la mesure du glucose peut être effectuée au moyen d'un capteur spécifique agissant sur les larmes voire la salive, et ce au moyen de dispositifs microélectroniques adéquats.

### L'empreinte digitale

Outre la mise en oeuvre sécurisée du système (tablette, PC, ou Smartphone) la prise de l'empreinte permet de sécuriser les « prélèvements » physiologiques effectués tels l'ECG ; adaptation qui éviterait la connexion d'un « intrus » sur les programmes.

L'on peut également procéder à la capture de données physiologiques pertinentes dans le but d'effectuer un suivi de l'apnée du sommeil.

### 2. Description d'un second mode de réalisation : un ordinateur portable adapté pour une collecte systématisée de données ECG etc...

L'on décrit à présent, en relation avec la figure 2 un second mode de réalisation de l'invention prenant la forme d'un ordinateur portable 590 adapté de manière à incorporer une centrale de diagnostique comportant au moins un capteur permettant de suivre une donnée physiologique de l'utilisateur au fur et à mesure que ce dernier se sert du dispositif pour ses activités quotidiennes, et notamment le pouls, la tension, la température, la pression de saturation en oxygène, P.H CT° etc....

En particulier, on dispose d'un capteur infrarouge ou photocapteur 592 (sur la figure 2) permettant de saisir le rythme cardiaque ainsi que la saturation d'oxygène et ce, au moyen d'une analyse de la vitesse de déplacement des globules rouges.

De cette manière, l'utilisateur du système peut disposer, alors qu'il consulte ses courriels ou travaille sur ses dossiers, d'informations pertinentes concernant sa pression, sa fréquence cardiaque...De manière préventive, le logiciel de diagnostic présent dans le système peut vérifier au jour le jour que les données recueillies restent confinées dans des valeurs acceptables et que cet utilisateur ne fait pas l'objet, par exemple, d'extrasystoles.

Dans un autre mode de réalisation, le photo capteur servant à cette analyse est combiné avantageusement avec un capteur qui est conventionnellement présent sur le système, à savoir un capteur d'empreinte digitale 591. De préférence, l'on dispose le capteur infrarouge 592 à proximité du capteur d'empreinte digitale 591 de manière à permettre, en une seule opération, la saisie de l'empreinte digitale - nécessaire au démarrage du système - mais également la saisie de la courbe de pression et de fréquence cardiaque ainsi que la mesure de la saturation d'oxygène.

Ainsi, dans la continuité d'un « même geste » , l'utilisateur peut satisfaire aux conditions de sécurité permettant le démarrage du système tout en obtenant des données médicales d'un grand intérêt pour lui.

De cette manière l'on obtient une combinaison unique et particulièrement avantageuse des fonctions de « sécurité » propres au système 590 et de fonctions de diagnostic médicales toutes aussi utiles pour l'utilisateur de ce même système.

Dans un autre mode de réalisation, l'on dispose en outre sur le dispositif 590 un jeu de deux électrodes bipolaires, respectivement gauche et droite, permettant une saisie de potentiel dans une configuration bipolaire. Une telle disposition permet alors au dispositif 590 et au logiciel de diagnostic médical en particulier de réaliser, au démarrage de la machine ou à tout moment opportun, une saisie de données pour la génération d'un électro-cardiogramme ECG.

De préférence, l'une des électrodes (droite par exemple) est disposée à proximité du capteur infrarouge ou même, comme cela est illustré dans la figure 2, est commune avec le capteur d'empreinte digitale 591 de manière à permettre la saisie au moyen du même doigt (droit) servant à la prise d'empreinte digitale et à la prise du rythme cardiaque, tandis que l'autre électrode (gauche) 593 se situe dans un emplacement à gauche du dispositif.

De manière générale, le logiciel de diagnostic procède à l'enregistrement périodique de données médicales de l'utilisateur du système au sein d'un espace mémoire protégé, par exemple dans une clé USB médicale, en fonction d'événements prédéterminés.

Ces données pourront servir en combinaison d'autres informations médicales générales saisies par l'utilisateur et susceptibles de constituer des facteurs de risque (age, poids, antécédents familiaux, fumeur ou non etc...) en réponse à un questionnaire généré automatiquement par le logiciel de diagnostic et auquel répond l'utilisateur.

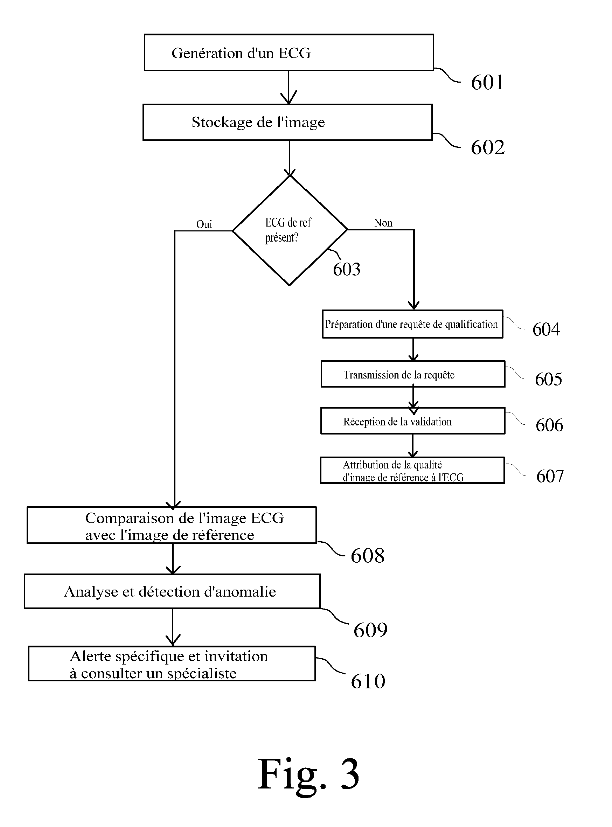

Dans un mode de réalisation particulier, outre le stockage des ECG générés périodiquement, le logiciel de diagnostic procède à une vérification périodique de l'électrocardiagramme généré automatiquement grâce aux deux électrodes bipolaires. A cet effet, une image de référence est générée par le logiciel, notamment lors de la première utilisation du dispositif par l'utilisateur, et qualifiée comme telle au moyen d'une procédure spécifique que l'on décrit à présent en relation avec la figure 3.

Le procédé de la figure 3 démarre avec une **étape 601** au cours de laquelle, lors du démarrage de l'ordinateur portable/tablette, une image ECG est générée au moyen des deux électrodes bipolaires présentes sur le système, comme cela a été décrit précédemment. D'une manière générale, le traitement analogique (amplification/filtrage) et numérique (conversion analogique/numérique) permettant la génération d'une telle image est bien connue d'un homme du métier et ne sera pas décrite plus avant.

Puis, dans une **étape 602,** le procédé procède au stockage de cette image ECG. Dans un mode de réalisation particulier, cette image ECG est stockée dans une zone spécifique de la mémoire du système, voire même une clé USB à vocation médicale, de manière à constituer une base de données particulièrement nourrie et utile pour le propriétaire du dispositif.

Puis le procédé détermine, au moyen d'un test dans une **étape 603,** si une image, qualifiée comme image de référence est déjà présente dans le système.

Si une telle image de référence est présente dans le système, le procédé poursuit directement avec une étape 608.

Dans le cas contraire, en l'absence d'une image de référence, le procédé poursuit avec **la préparation d'une requête de qualification** au cours d'une **étape 604.** A cet effet, le procédé procède à la préparation d'une requête intégrant l'image ECG saisie avec des informations supplémentaires, par exemple administrative (nom de l'utilisateur) voire mêmes médicales. Optionnellement, cette étape de préparation pourra suivre une phase préliminaire de validation « interne » de l'image ECG par le logiciel de diagnostique de manière à s'assurer que l'image ECG obtenue lors de l'étape 601 présente un niveau de qualité suffisant pour pouvoir être utilisée dans la phase de préparation de l'étape 604.

Dans un mode de réalisation particulier, afin de renforcer la sécurité et la pertinence du diagnostic qui sera ultérieurement effectué par le logiciel de diagnostic, l'image ECG qui est soumise à des fins de validation est signée au moyen de données d'identification appartenant à l'utilisateur.

L'on pourra par exemple avantageusement associer l'image ECG capturée lors de l'étape 601, alors que l'utilisateur avant posé deux doigts sur les électrodes bipolaires, avec une image prise par la caméra (*webcam*) au moment de la capture de l'ECG ou, alternativement (voire cumulativement), avec l'empreinte digitale saisie simultanément ou conjointement au moyen du capteur d'empreinte digitale.

Ainsi, l'on parvient à associer irrémédiablement une image ECG - qui pourra être ultérieurement qualifiée comme image de référence, avec une donnée d'identification (empreinte digitale) particulièrement sûre et qui permettra au cardiologue ou à l'expert validant la qualification de conserver en même temps l'image ECG qu'il aura validé avec l'empreinte digitale du doigt qui aura servi à la génération de cette image. La sécurité de cette association entre l'image ECG et l'empreinte digitale pourra être renforcée d'avantage par divers moyens de signatures, de hashage bien connus d'un homme du métier.

D'une manière pratique, une fois la requête préparée lors de l'étape 604, celle-ci pourra aboutir à différents formats, comme par exemple un fichier XML intégrant l'image JPG de l'électrocardiogramme, voire même un message électronique. Le fichier XML pourra d'ailleurs intégrer toute autre donnée médicale susceptible d'être collectée par le dispositif 590.

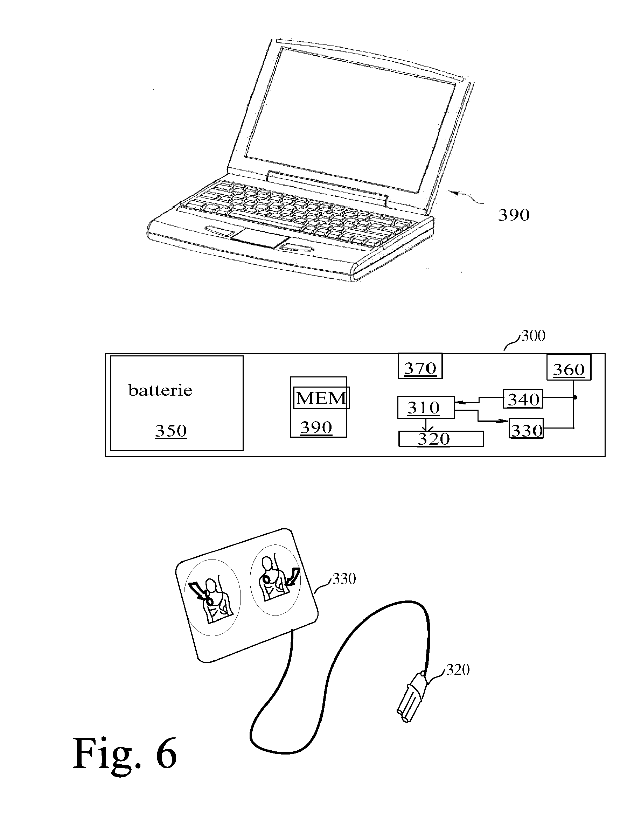

Puis, dans une **étape 605,** le procédé mis en oeuvre par le logiciel de diagnostic procède à l'envoi de la requête de qualification vers un serveur distant spécialisé (non illustré dans la figure 6).

D'une manière générale, la transmission de la requête de qualification pourra utiliser tous les moyens modernes de télétransmission, filiaires, sans fils, et s'appuyant notamment sur les protocoles de transmission (IP) en usage dans le réseau Internet.

Par ailleurs, le serveur distant est un serveur spécialisé, géré par des experts compétents dans le domaine de la cardiologie et pouvant valider l'image ECG reçue lors de l'étape 605.

En pratique, l'on pourra également prévoir que le serveur distant sera un système informatique détenu par le cardiologue habituel de l'utilisateur du systeème, lequel pourra alors examiner à loisir l'image ECG générée par le dispositif 590 et valider la qualification d'image de référence. Dans l'hypothèse où l'image ECG est signée par des données d'identification de l'utilisateur, la validation par le cardiologue traitant permettant à ce dernier de s'assurer que l'image de référence ne pourra êtte détournée et utilisée par un autre système de diagnostic que celui qui apparaît à son utilisateur.

Cette validation de la qualification est alors reçue au cours d'une **étape 606** par le dispositif 590 qui peut alors, dans une **étape 607,** stocker définitivement l'image ECG avec la qualification d'image de référence. Cette image pourra alors servir à des fins de comparaison, notamment lors d'une analyse des images ECG qui seront générées quotidiennement lors de l'utilisation du dispositif 590.

En effet, à la suite du test de l'étape 603, si le procédé a conclu en la présence d'une image de référence stockée au sein du système, celui-ci procède ensuite, dans une **étape 608,** à une comparaison de l'image ECG nouvellement acquise avec cette image de référence.

Cette comparaison se poursuit ensuite, lors d'une **étape 609,** par une analyse circonstanciée de l'image ECG nouvellement acquise de manière à permettre, le cas échéant, la détection d'anomalie particulières, mises en évidence lors de la comparaison de l'étape 608. Dans un mode de réalisation particulier, le procédé procède au monitoring des systoles, des arythmie et toute anomalie susceptible d'être détectée sur l'ECG.

Si une telle anomalie est détectée, alors le procédé poursuit, lors d'une étape 610, par la génération d'une alerte spécifique destinée à l'utilisateur du dispositif 590, voire même, le cas échéant, au serveur distant.

Le système qui vient d'être décrit présente un grand intérêt d'un point de vue médical pour l'utilisateur de l'ordinateur portable ou de la tablette 590. En effet, sans que celui-ci s'en préoccupe vraiment, il lui est offert, grâce aux dispositifs et procédures décrits précédemment, la possibilité de générer automatiquement une image ECG de référence mais également des images périodiques qui pourront être utilement comparées à l'image de référence dans le but de détecter diverses anomalies et situation de risque cardiaque. Ainsi, six mois après avoir acquis le système et généré sa première image ECG (supposée normale), le logiciel de diagnostic pourra être en mesure de l'alerter sur l'existence de divers problèmes apparaissant sur l'ECG, par exemple, une anomalie de la repolarisation, une anomalie de la contraction auriculaire etc...

Ainsi prévenu et alerté , l'utilisateur pourra consulter rapidement.

En particulier, certaines situations particulièrement préoccupantes pourront être prévenues, notamment la situation d'infarctus silencieux qui, comme cela est connu, ne se manifestent pas par les symptômes habituellement rencontrés (mal dans le bras, mal de mâchoire, étau dans la poitrine). Le patient susceptible d'en souffrir ne peut ainsi aisément détecter le risque (autrement qu'en allant consulter régulièrement son cardiologue). En revanche, la situation d'infarctus silencieux se détecte de manière électrique grâce à une analyse de l'ECG, comme l'analyse faite lors de l'étape 609 de la figure 6. Ainsi, sous réserve qu'il utilise suffisamment fréquemment son système (notamment pour accéder à ses courriels), l'utilisateur peut être prévenu assez tôt qu'il présente un risque important ou, même, qu'il a fait un infarctus silencieux ... Face à un tel risque, il peut alors aller consulter rapidement. Cet exemple montre ainsi le grand intérêt de la présente invention.

Dans des modes de réalisation particuliers, le dispositif 590 pourra être enrichi de plusieurs fonctionnalités de diagnostique supplémentaires qui pourront utilement venir compléter l'image ECG :
- la prise de pression artérielle au moyen d'un brassard adapté, par exemple pour être connecté par un port USB sur le dispositif 590 ;
- des données relatives au monitoring de l'apnée du sommeil . A cet effet, le dispositif 590 comportera un port permettant la connexion de deux électrodes supplémentaires placés sur le thorax ou, alternativement, permettra une connexion par des moyens sans fil à un dispositif autonome auquel seront connectées ces deux électrodes. D'une manière générale, la prise de données caractéristiques de monitoring de l'apnée du sommeil est bien connu d'un homme du métier et il n'est pas besoin d'alourdir l'exposé avec des détails supplémentaires.
- Le contrôle de la glycémie au moyen d'un appareil adapté à cet effet et susceptible d'être connecté par des moyens de communications (port USB ou moyen sans fil) au dispositif 590.
- Le contrôle de la courbe de poids au moyen d'une balance adéquate susceptible de communiquer avec le logiciel de diagnostique.

### 3. Description d'un troisième mode de réalisation d'un dispositif de collecte de données physiologique communiquant avec un défibrillateur

Dans les modes de réalisation qui vont suivre, l'on combine avantageusement les fonctionnalités offertes par la centrale de diagnostique décrite précédemment, avec les fonctionnalités d'un défibrillateur, plus ou moins intégré, tel que décrit dans la demande de brevet n°12/02189 en date du 6 Août 2012, et non publiée à la date de dépôt de la présente demande de brevet.

Un tel défibrillateur s'avère particulièrement avantageux pour accroître la présence d'un dispositif, du fait de la grande disponibilité des ordinateurs/tablettes sur les lieux publics. En effet, les statistiques du ministère de la Santé font état de 50000 décès chaque année dus à des accidents cardio-respiratoires. Dans presque la moitié des cas, ces accidents sont provoqués par une fibrillation ventriculaire initiale qui, en l'absence d'un traitement adéquat appliqué dans les deux ou trois minutes, entraîne la mort. Les accidents cardio-respiratoires peuvent en effet apparaître n'importe où et frapper n'importe qui, que celui-ci se trouve dans son environnement professionnel, en train de faire des courses, sur le lieu de ses loisirs ou au restaurant.

Lorsque intervient l'accident, l'urgence est immédiate et il faut pouvoir assurer un secours immédiat même en l'absence d'un médecin ou d'un urgentiste.

La centrale de diagnostique qui vient décrite peut ainsi être significativement améliorée en venant introduire un défibrillateur directement au sein du dispositif de traitement de données.

De cette manière, l'on accroît significativement la possibilité pour tout un chacun de trouver, dans une situation d'urgence où l'espérance de vie se mesure en moins d'une dizaine de minutes, un dispositif de défibrillation complet capable d'être mis en oeuvre d'une manière simple et ce notamment par un opérateur non professionnel qui ne dispose pas des compétences, des connaissances et du sang froid des professionnels de la santé.

A cet égard, afin d'accroître la simplicité de la solution préconisée, l'on propose la mise en oeuvre d'un dispositif défibrillateur présent, de fabrication, au sein du dispositif de traitement de l'information mobile, de manière à combiner avantageusement les avantages résultant de la capture de données physiologiques (propre à l'utilisateur) aux fonctions du défibrillateur qui, elles, peuvent bénéficier non seulement à ce dernier mais non exclusivement.

En permettant à un opérateur - par hypothèse non professionnel - de bénéficier des fonctionnalités multimédias d'un système d'information mobile, qu'il s'agisse d'un ordinateur portable, d'une tablette tactile, d'un téléphone intelligent (désigné dans la littérature anglo-saxonne sous le vocable "*smartphone*"), d'un assistant numérique personnel (PDA) l'on accroît ainsi considérablement non seulement la **DISPONIBILITE** de ces défibrillateurs mais également leur **SIMPLICITE** d'utilisation et, par conséquent, les perspectives de survie d'une victime potentielle d'un accident cardio-respiratoire.

Tout en préservant, comme on va le voir plus loin, la **SECURITE** de l'opération, grâce aux multiples aménagements qui seront décrits ci-après dans les différents modes de réalisation.

Dans un mode de réalisation préféré, le dispositif de traitement de données assurant la collecte de données physiologiques comporte, de fabrication, un défibrillateur de manière à offrir des fonctionnalités utiles et d'un grand intérêt qui coopèrent les unes avec les autres.

Mais cela n'est qu'un possible de l'invention, et le débrillateur pourra prendre clairement la forme d'un dispositif périphérique, venant coopérer au moyen d'une communication adéquate avec le dispositif de traitement de données.

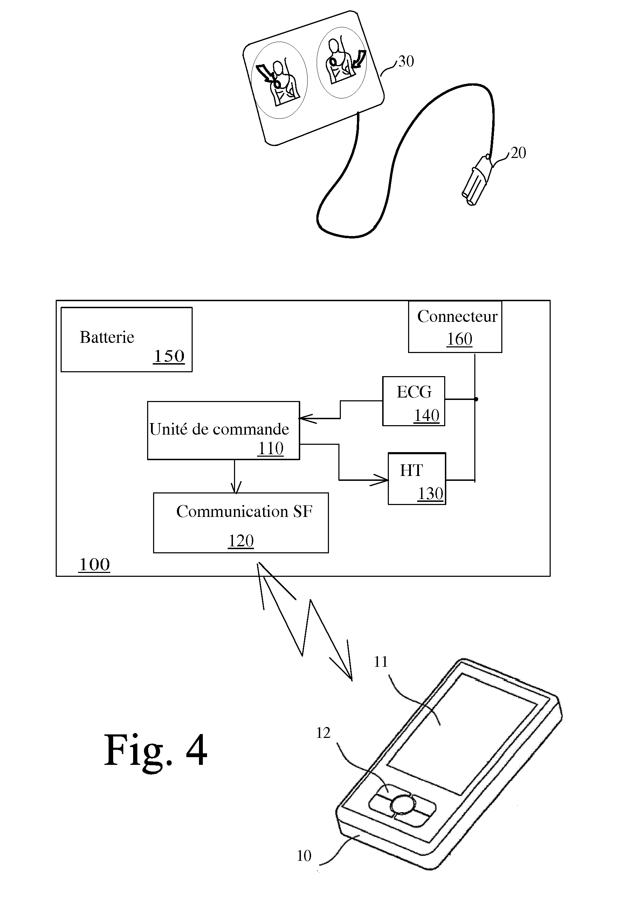

La **figure 4** illustre une telle réalisation où l'on voit, dans le troisième mode de réalisation, que le dispositif prend la forme d'un téléphone intelligent ou « smarphone », proprement adapté pour permettre une collecte systématisée d'information physiologiques comme cela a été décrit précédemment, et dans lequel on met à profit les fonctionnalités de communication de celui-ci pour assurer la communication avec un dispositif défibrillateur périphérique.

D'une manière générale, l'architecture interne d'un dispositif défibrillateur et la structure de ses circuits internes sont bien connues d'un homme du métier et il n'est pas nécessaire d'alourdir l'exposé par une description exhaustive de ces circuits.

Comme cela est illustré dans la figure 4, l'on considère un défibrillateur, par exemple automatisé externe (DAE) 100 se présentant sous la forme d'un boîtier comportant un connecteur 160 destiné à recevoir un connecteur correspondant 20 relié à une paire d'électrodes 30. Le défibrillateur 100 comporte un bloc fonctionnel 140 destiné à l'analyse et au traitement du signal ECG (Electrocardiogramme) capté par les électrodes 30 ainsi qu'un bloc fonctionnel 130 apte à délivrer un choc électrique Haute-Tension (HT) de plusieurs centaines de volts et plusieurs centaines de Joules.

Le défibrillateur comporte en outre une unité de commande 110 commandant les blocs fonctionnels 130 et 140 ainsi qu'une unité de communication sans fil 120 permettant la communication sans fil avec un dispositif de traitement de données externe 10, comme le téléphone mobile représenté dans la figure. Dans un mode de réalisation particulier, la communication entre le boîtier 100 et le téléphone mobile 10 pourra être basé sur le protocole WIFI et notamment les divers protocoles de communication sans fil régis par les normes du groupe IEEE 802.11 (ISO/CEI 8802-11), mais aussi *Bluetooth,* etc... A titre d'exemple, le téléphone intelligent pourra être un téléphone fonctionnant sous un système d'exploitation open source utilisant le noyau Linux, de type Android. Il est clair cependant qu'un homme du métier pourra adapter l'invention à tout autre système d'exploitation, voire tout autre type de téléphone mobile, smarphones, tablettes tactiles, PDA et terminaux mobiles etc...

Comme cela est connu d'un homme du métier, le bloc fonctionnel ECG 140 sert à capter le signal des électrodes 30 en vue d'une analyse automatique de l'activité électrique du myocarde d'une personne victime d'un arrêt circulatoire afin de déceler une fibrillation ventriculaire ou certaines tachycardies ventriculaires. Lorsque l'analyse s'avère être positive, l'unité de commande 110 peut activer le chargement de l'appareil - et notamment d'un ensemble de condensateurs internes disposés dans le bloc HT 130, et délivrer les chocs électriques externes transthoraciques, d'intensité appropriée, dans le but de rétablir une activité circulatoire normale.

Le défibrillateur 100 est alimenté par une batterie autonome 150 assurant l'alimentation électrique de tous les circuits électroniques du défibrillateur et notamment la fourniture de l'énergie nécessaire à la délivrance des chocs électriques diffusés par la paire d'électrodes 30.

Le dispositif 100 illustré dans la figure 4, et l'unité de commande 110 qu'il comporte sont adaptés à une communication avec le téléphone portable 10 suivant un mode lui permettant de bénéficier des fonctionnalités multimédia - en particulier audio et vidéo - permettant à tout premier secours - même lorsqu'il ne s'agit pas d'un professionnel de la santé - de bénéficier d'un assistant multimédia de haut niveau qui s'avère particulièrement préciser pour l'aider dans la mise en oeuvre du défibrillateur.

A cet égard, lors de l'activation du défibrillateur 100, les fonctionnalités du téléphone mobile 10, notamment l'écran (tactile ou non) 11, l'écran 12 et les fonctions audio, seront toutes réservées au fonctionnement du défibrillateurs de manière à assurer, avec une sécurité maximale le fonctionnement du défibrillateur et la délivrance des chocs électriques.

Plus spécifiquement encore, cette réservation exclusive des fonctionnalités du dispositif mobile seront automatiquement assurées dès le branchement des électrodes au système, via leur connecteur spécifique.

Dès lors qu'interviendra le branchement physique des éléments, un logiciel d'application ou un jeu de micro instructions mises en oeuvre dans le microprocesseur du téléphone mobile 10 assureront la coopération de ce dernier dans les trois phases majeures du fonctionnement du défibrillateur 100:
phase 1: la mise en fonctionnement du défibrillateur, l'activation de ses circuits internes et la mise en place de la paire d'électrodes 20;
phase 2: l'analyse et le traitement du signal ECG perçu par la paire d'électrodes 30 en vue d'identifier une situation de fibrillation ventriculaire; et
phase 3: la délivrance d'un choc électrique lorsque l'analyse de la phase 2 s'avère positive.

Pendant toute la durée de ces trois phases, l'opérateur bénéficie ainsi d'instructions audibles et visuelles particulièrement claires qui lui sont transmises par le téléphone mobile, le *smartphone*, le PDA etc... communiquant avec le défibrillateur 100, accroissant ainsi l'efficacité et la sécurité de son intervention.

D'une manière générale, l'installation du logiciel d'application ou microprogramme au sein du système d'exploitation du téléphone 10 pourra s'effectuer de manière bien connue, notamment à la suite d'une phase de téléchargement de ce même logiciel depuis une zone de stockage située dans défibrillateur 100, par exemple via les moyens de communication sans fil 120. Dans cette hypothèse, il est à noter que la phase d'installation aura été effectuée préalablement à la mise en oeuvre du défibrillateur, ce qui exclut évidemment une situation d'urgence.
On décrira dans la suite, des modes de réalisation où l'on pourra notamment assurer la mise en oeuvre du défibrillateur même dans une situation d'urgence.

De manière générale, on pourra prévoir également que le logiciel ou le microprogramme installé au sein du téléphone 10 pourra servir également à des opérations de maintenance et de contrôle du défibrillateur, notamment du contrôle du niveau de charge de la batterie interne 150.

Dans un mode de réalisation particulier, le logiciel d'application du défibrillateur provoquera un séquencement de phases d'éveil du défibrillateur pour vérifier de manière périodique l'état de charge de la batterie ainsi que les systèmes électroniques critiques de ce dernier.

### 4. Description d'un quatrième mode de réalisation : une tablette tactile communiquant avec un défibrillateur

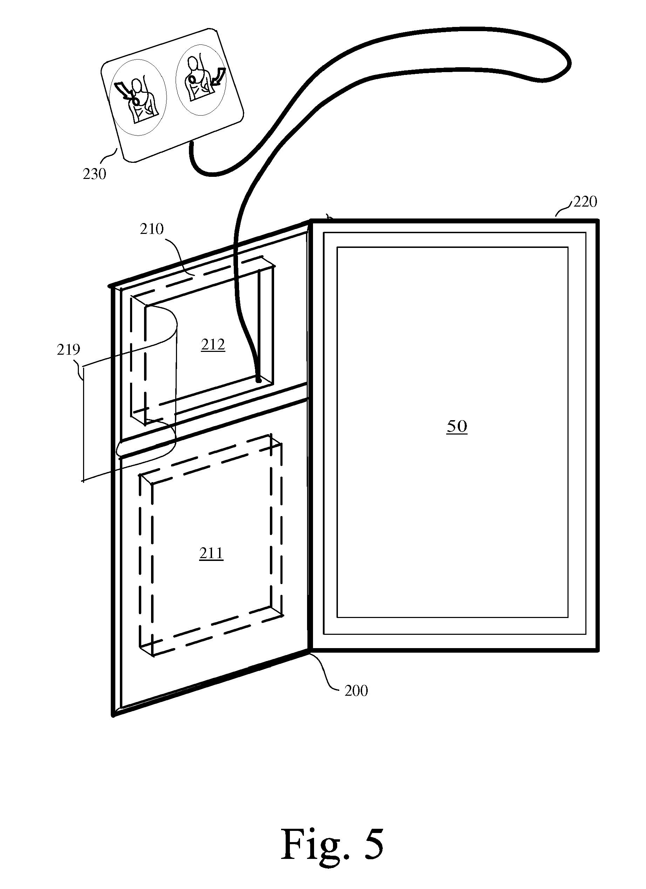

On décrit à présent en relation avec la figure 5 un quatrième mode de réalisation dans lequel le dispositif de traitement de données permettant la capture de données physiologiques prend la forme d'une "tablette tactile", communiquant avec un défibrillateur. On comprendra dans cette acception tous les dispositifs se présentant sous la forme d'un panneau d'affichage et non dotés d'un clavier mécanique comme ceux qui équipent les ordinateurs portables.

La figure 5 illustre une tablette tactile 50 comportant une housse ou coque se présentant sous la forme d'une couverture 200 qui pourra être en tout matériau plastique, cuir, flexible ou non etc...

De préférence, la couverture 200 se présente, à l'instar de celle d'un livre, avec un volet gauche (ou recto) et un volet droit (verso), ce dernier comportant des moyens de fixation de la tablette tactile 210.
Ainsi, l'ensemble couverture-tablette forme l'équivalent d'un *livre* protégé par la couverture 200 comportant les deux volets recto 210 et verso 220.

Suivant un mode de réalisation de la présente invention, l'élément recto 210 présente une certaine épaisseur, par exemple inférieure à 1 cm, et comporte au moins un compartiment interne - comme le compartiment inférieur 211 de la figure 2, destiné à recevoir les circuits électroniques d'un défibrillateur 200 semblable au défibrillateur 100 qui avait été décrit en relation avec la figure 1.

Les progrès de la miniaturisation, notamment l'emploi de condensateurs au tantale permettent d'envisager la mise en place des blocs fonctionnels 210, 230, 240 (équivalents aux blocs 110, 130 et 140 de la figure 1), ainsi qu'un bloc de batterie lonlithium permettant l'alimentation électrique du défibrillateur.

De préférence, le volet gauche 211 comporte en outre, sur sa partie supérieure, un second compartiment 212 destiné à recevoir une paire d'électrodes 230 semblables aux électrodes 30 illustrées dans la figure 1.

De préférence, les électrodes 230 ne sont accessibles depuis le compartiment 212 qu'au moyen d'un accès scellé assurant un usage unique du défibrillateur de manière à accroître la sécurité de fonctionnement, comme ce que l'on a représenté dans la figure 2 par la déchirure d'une pellicule 219, qui empêchait jusqu'alors l'accès au compartiment 212 et qui est rabattue à gauche du volet recto 210.

Grâce à la mise en place de ce scellé 219, l'on assure l'intégrité du défibrillateur pour une unique utilisation en toute sécurité, et l'on prévient en particulier une décharge inopportune de la batterie contenue dans le compartiment 211 du volet gauche (recto).

De la même manière que précédemment, préalablement à l'utilisation du défibrillateur contenu dans le compartiment 211, l'on vient installer par des moyens appropriés un logiciel d'application dans le système d'exploitation de la tablette tactile 50 de manière à permettre notamment la communication sans fil avec le défibrillateur mais également diverses fonctions de maintenance et de contrôle, notamment de contrôle épisodique régulier (tous les mois par exemple) du niveau de charge de la batterie interne stockée dans le compartiment 211. Dans un mode de réalisation particulier, le logiciel installé procède à la vérification de l'intégrité du scellé 219, par tout système quelconque, tel que par exemple un fil de rupture, un détecteur capacitif ou de lumière etc....

Grâce au concours du défibrillateur disposé dans le compartiment 211 et des fonctionnalités multimédias mises à disposition par la tablette tactile, l'utilisateur premier secours tirer avantage des fonctionnalités audio-vidéo et bénéficier d'un tutoriel ou d'une assistance précieuse lors de la mise en oeuvre du défibrillateur.

De préférence, le logiciel mis en oeuvre dans la tablette tactile détecte la rupture du scellé 219 et met en oeuvre les fonctionnalités de géo-positionnement de manière à transmettre un message d'alerte à une équipe de secours permettant ainsi à l'opérateur premier secours de se concentrer sur la mise en oeuvre du défibrillateur, tout en sachant qu'une équipe professionnelle est appelée à se rendre immédiatement sur les lieux.

A cet égard, le logiciel pourra utiliser les fonctionnalités de télécommunications avancées présentes dans la tablette tactile, notamment les accès GSM, GPRS, 3G et 4G potentiellement présentes dans le système.

Ainsi, en même temps que l'utilisateur met en oeuvre le défibrillateur situé dans le compartiment 211, un message d'alerte, généré automatiquement dès la rupture du scellé 219 est transmis à un centre d'appel adéquat, accroissant ainsi l'efficacité du système de secours.

Dans un mode de réalisation particulier, le volet gauche 210 comporte en outre un connecteur USB ou micro-USB (Universal Serial Bus) permettant la connexion à la tablette tactile ou à l'assistant digital portable (PDA) et pouvant servir, soit à l'installation d'un logiciel adéquat au sein de cette dernière, soit la recharge ou le maintien en charge de la batterie autonome stockée dans le compartiment 211.

Dans cette dernière configuration, l'on peut également prévoir que la batterie présente dans le compartiment 211 puisse également servir de batterie de secours pour la tablette tactile 50 grâce à son port USB. Dans ce cas, le logiciel ou microprogramme installé dans le système d'exploitation de cette batterie assurera le suivi de l'état de charge et de décharge de cette batterie et générera les alertes à l'attention de l'utilisation pour l'inviter à recharger dès que possible la batterie du défibrillateur. Dans cette configuration, l'on pourra avantageusement prévoir des circuits électroniques permettant de tracer les phénomènes de charge/décharge de la batterie pour permettre de suivre précisément les conditions de fonctionnement et d'utilisation de cette dernière. Les informations adéquates pourront être notamment transmises au fabricant du défibrillateur par le logiciel d'application s'exécutant sur la tablette tactile au moyen des outils de communication mobiles (WIFI, GSM, GPRS, 3G, 4G etc..)

L'on voit sur les exemples qui sont décrits à quel point peut être fertile et avantageuse la combinaison des circuits du défibrillateur avec un téléphone mobile et/ou une tablette tactile.

Mais comme on va le voir à présent avec le troisième mode de réalisation, c'est avec un ordinateur portable, que la coopération des systèmes apparaît la plus féconde.

### 5. Description d'un cinquième mode de réalisation : un ordinateur portable doté d'un pack batterie comportant un défibrillateur

Dans le cinquième mode de réalisation qui est décrit à présent en référence avec la figure 6 le défibrillateur est d'emblée installé dans un pack de batterie destiné à correspondre à un modèle spécifique d'un ordinateur portable 390 d'une marque connue, et également adapté pour permettre la collecte systématisée de données physiologiques comme cela a été décrit précédemment.

L'on pourra ainsi prévoir de couvrir, avec des circuits électroniques communs, une large gamme d'ordinateurs portables.

En se référant à la figure 6, l'on voit que le pack batterie/défibrillateur 300 comporte, outre une batterie autonome 350, un connecteur 360 destiné à permettre le branchement d'un connecteur 320 d'une paire d'électrodes 330.

Dans un mode de réalisation particulier, le boîtier du pack batterie/défibrillateur 300 comporte un compartiment permettant de loger la paire d'électrodes 330 et son connecteur 320.

Le pack batterie/défibrillateur 300 comporte en outre un bloc fonctionnel 340 destiné à l'analyse et au traitement du signal ECG ainsi qu'un bloc fonctionnel 330 apte à délivrer le choc électrique HT. Il comporte également une unité de commande 310 commandant les blocs fonctionnels 330 et 340.

Optionnellement, le block batterie/défibrillateur comporte également une unité de communication sans fil 320 permettant une éventuelle communication sans fil, soit avec l'ordinateur portable, soit avec tout autre système de traitement de données.

D'une manière générale, l'ordinateur portable 390 comporte un système d'exploitation quelconque, tel que notamment Windows XP ou Windows 7 de l'éditeur MICROSOFT Corp. Alternativement, l'on pourra envisager un système d'exploitation de type LINUX.

Dans un mode de réalisation particulier, le bloc de commande 310 communique avec le système d'exploitation de l'ordinateur portable de manière à permettre au défibrillateur de bénéficier des fonctionnalités multimédias de ce dernier.

Dans un mode de réalisation préféré, pour éviter tout risque afférent au système d'exploitation s'exécutant dans l'ordinateur portable, l'on prévoit au sein du défibrillateur 300 une zone mémoire 390 comportant un microprogramme de démarrage , écrit en un langage quelconque, permettant de prendre en charge toute la procédure de démarrage de l'ordinateur portable grâce à un connecteur USB connecté à un port USB 370.

De cette manière, l'on court-circuite l'exécution du système d'exploitation pour assurer que la totalité des ressources de l'ordinateur portable sont bien effectivement affectées au fonctionnement du seul défibrillateur 300, et ce dès le branchement des électrodes sur leur connecteur dédié.

A cet effet, dans un mode de réalisation particulier, nullement limitatif, le pack batterie/défibrillateur 300 de la figure 6 comporte des circuits de commutation spécifiques pour permettre une commutation quasi immédiate sur le logiciel du défibrillateur .

A cet égard, le dispositif 300 comporte des circuits de commutation détectant la connexion de la paire d'électrodes 330 sur le connecteur 360, dans le but de provoquer une rupture d'alimentation de l'ordinateur portable 400. Créant de ce fait, d'une manière sans doute brutale mais particulièrement efficace, l'extinction du système d'exploitation qui est en cours d'exécution.

En outre, le pack batterie/défibrillateur comporte des systèmes de commutation supplémentaires (intégrés dans le module de commande 310) pour n'autoriser l'alimentation électrique de l'ordinateur qu'à condition que le port USB 370 n'est branché sur un des ports USB de l'ordinateur portable.

Cette disposition, permet d'assurer un redémarrage de la machine, hors système d'exploitation, sur un microprogramme stocké dans la mémoire 390 localisée dans le pack batterie/défibrillateur 300, sous réserve de la configuration adéquate préalable de la séquence de "*boot*" du BIOS (*Basic Input Output Sequence*) de la carte mère de l'ordinateur portable.

De ce fait, l'on parvient avantageusement à éviter que ne se mettent en oeuvre les procédures de test du système d'exploitation à la suite de l'arrêt brutal de ce dernier, et qui sont clairement incompatibles avec le démarrage du défibrillateur 300.

On parvient ainsi à éviter au premier secours la perte d'un temps crucial.

De préférence le microprogramme est un logiciel développé en Linux, ou voire même en DOS (*Disk Operating System*) permettant un démarrage ultra-rapide.

Alternativement, l'on pourra recourir à tout autre langage , tel que le C++, lequel peut servir au fonctionnement d'un ordinateur dénué de tout système d'exploitation. L'homme du métier se reportera notamment aux derniers développement en matière de programmation hors système d'exploitation (désigné dans la littérature anglo-saxonne par l'appellation "*bare computing*"). Voir notamment la référence:
*"How to run C*++ *applications on a bare PC*?" de Dr Ramesh K. Karne et al,.

L'on assure ainsi un démarrage quasi immédiat de l'ordinateur portable, avec l'assurance que toutes les ressources de l'ordinateur seront réservées au seul défibrillateur 300. Le premier secours est alors assuré de ce qu'il pourra bénéficier des ressources multimédias, notamment audio-vidéos mais également des fonctionnalité de géolocalisation et de communications mobiles lui permettant de bénéficier alors d'un tutoriel, d'une assistance efficace et précieuse pour assurer un fonctionnement approprié du défibrillateur 300 .

### 6. Description d'un sixième mode consistant en un ordinateur portable doté d'un défibrillateur logé au sein d'un caddie

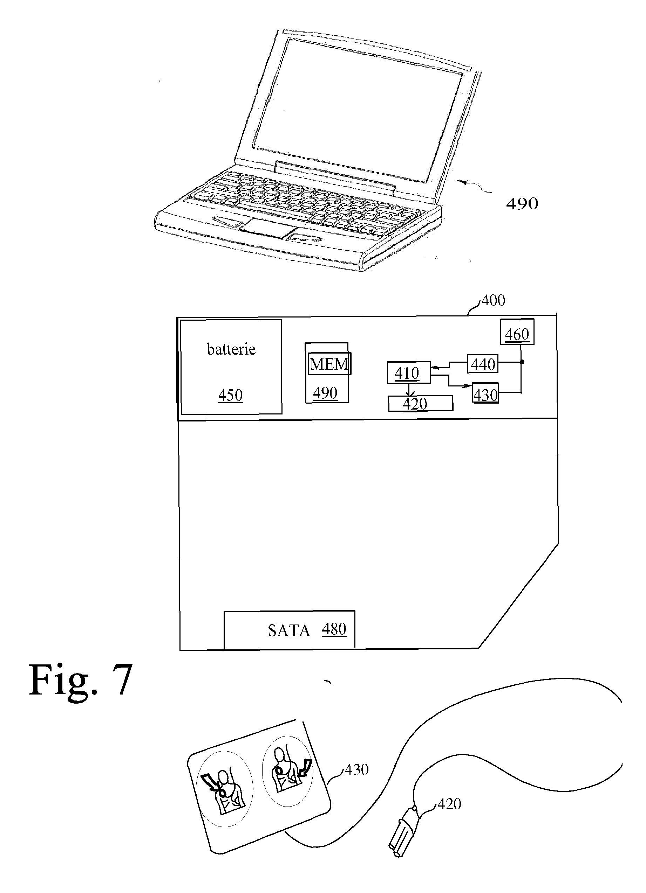

Dans le sixième mode de réalisation qui est décrit à présent en relation avec la figure 7, l'on voit que le défibrillateur se présente sous la forme d'un "caddie" 400 destiné à occuper la place d'un lecteur de CDR/DVD d'un ordinateur portable. En particulier, le "*caddie*" comporte un port SATA 480 destiné à venir se connecter sur le port correspondant de la carte mère de l'ordinateur portable.

D'une manière similaire au pack batterie/défibrillateur de la figure 6, le défibrillateur 400 comporte une batterie autonome 450 (qui pourra être de capacité plus réduite que la batterie 350 de la figure 3 puisqu'elle n'est pas destinée à servir à l'alimentation électrique de l'ordinateur portable), et un connecteur 460 destiné à permettre le branchement d'un connecteur 420 d'une paire d'électrodes 430. De préférence, la paire d'électrodes 430 pourra se loger spécifiquement dans un compartiment spécifique du "caddie" 400.

Le défibrillateur 400 comporte en outre, de manière similaire au défibrillateur 300 de la figure 6, un bloc fonctionnel 440 destiné à l'analyse et au traitement du signal ECG ainsi qu'un bloc fonctionnel 430 apte à délivrer le choc électrique HT. Il comporte également une unité de commande 410 commandant les blocs fonctionnels 430 et 440. Le défibrillateur 400 comporte également une zone mémoire 490 destiné à recevoir les micro instructions servant au démarrage de l'ordinateur portable, lequel pourra être effectué cette fois ci directement depuis le connecteur SATA 480 enfiché sur le bus correspondant de la carte mère.

Comme précédemment, optionnellement, le block batterie/défibrillateur comporte également une unité de communication sans fil 420 permettant une éventuelle communication sans fil, soit avec l'ordinateur portable, soit avec tout autre système de traitement de données.

D'une manière générale, l'ordinateur portable 490 comporte un système d'exploitation quelconque, tel que notamment Windows XP ou Windows 7 de l'éditeur MICROSOFT Corp. Alternativement, l'on pourra envisager un système d'exploitation de type LINUX ou tout autre système d'exploitation.

On pourra également prévoir, comme pour le mode de réalisation précédent, des circuits de commutations (intégrés dans le bloc 410) permettant de rendre actif le défibrillateur 400 dès lors que l'opérateur aura branché la paire d'électrodes. En particulier, les circuits de commutation pourront ne rendre actif la connexion via la port SATA 480 uniquement lorsque la connexion de la paire d'électrodes aura été effectué via le connecteur 460.

Pour assurer une disponibilité immédiate des ressources de l'ordinateur portable au seul défibrillateur 400, un mécanisme spécifique est prévu basé sur la détection du branchement des électrodes 430, de manière à sécuriser le fonctionnement de l'ordinateur lors de la mise en oeuvre du défibrillateur et éviter toute interruption dans le fonctionnement de ce dernier.

Une fois les ressources de l'ordinateur portable alloué au défibrillateur 400, l'opérateur premier secours dispose alors des fonctionnalités multimédias présentes dans l'ordinateur portable, lui assurant ainsi le support d'un assistant efficace lors de l'opération de défibrillation.

Comme on le voit , la présente invention permet une multitude de configurations permettant d'accroître la simplicité, l'efficacité ainsi que la sécurité du premier secours.

L'on notera en particulier que les modes de réalisation qui sont décrits ne sont nullement exclusifs les uns par rapport aux autres. En effet, comme on va le voir, le troisième mode de réalisation qui vient d'être décrit, outre qu'il permet le fonctionnement d'un ordinateur portable (en lui assurant son alimentation électrique) et également avec un ordinateur portable en faisant bénéficier le premier secours des fonctionnalités multimédias de l'ordinateur portable, on pourra également avantageusement prévoir que ce "pack batterie" qui vient d'être décrit puisse également fonctionner, grâce au bloc de communication 320, avec un téléphone mobile intelligent, ou une tablette tactile comme cela a été décrit en relation avec la figure 1.

L'on constate ainsi la grande possibilité d'adaptation de l'invention permettant, dans bien des situations d'urgence, d'avoir une aide effective et critique au premier secours d'une victime d'un accident cardio-respiratoire.

## Revendications

1. Dispositif de traitement de l'information de type ordinateur, ordinateur portable, tablette tactile et/ou téléphone intelligent comportant :
- au moins un capteur pour la capture d'une donnée physiologique de l'utilisateur dudit dispositif de traitement de l'information;
- des moyens d'activation dudit capteur pour procéder à la capture en réponse à un événement périodique donné;
- des moyens de stockage pour le stockage de la donnée physiologique capturée en association avec une information de datage et/ou une information relative à l'utilisateur et/ou audit événement.

2. Dispositif selon la revendication 1 **caractérisé en ce que** ledit événement correspond au démarrage du système en sorte que lesdits moyens périodiques provoquent un stockage automatique d'une donnée physiologique datée à chaque démarrage de la machine en vue de la constitution d'une base de données physiologique propres à l'utilisateur.

3. Dispositif selon la revendication 1 **caractérisé en ce que** ledit événement correspondant au lancement d'un programme informatique ou d'une routine logicielle de manière à constituer la réponse physiologique de l'utilisateur lors de l'utilisation dudit programme ou de ladite routine, ledit dispositif comportant des moyens pour différer le démarrage dudit programme ou de ladite routine logicielle tant que la collecte des paramètres n'a pas eu lieu.

4. Dispositif selon l'une des revendications précédentes **caractérisé en ce que** la ou les données physiologiques sont stockées sur un support amovible , par exemple de type clé USB, ou transférés sur un serveur distant.

5. Dispositif selon l'une des revendications précédentes comportant un capteur infrarouge est localisé à proximité immédiate d'un capteur d'empreinte digitale présent à des fins de sécurité, de manière à assurer conjointement :
- la saisie de la courbe de pression et de fréquence cardiaque en même temps que la prise d'empreinte digitale destinée à l'activation du système ;
- la signature des courbes de pression et de fréquence cardiaque par une information dérivée du capteur d'empreinte digitale de manière à associer irréversiblement les données saisies par le capteur infrarouge et l'empreinte digitale de l'utilisateur

6. Dispositif selon l'une des revendications précédentes **caractérisé en ce que** la donnée physiologique est une image de l'iris de l'oeil capturé au moyen d'une caméra présente disponible dans le système.

7. Dispositif selon l'une des revendications précédentes **caractérisé en ce que** la donnée physiologique est une empreinte vocale enregistrée par un dispositif audio présent dans le système, lorsque l'utilisateur répète une donnée prédéterminée.

8. Dispositif selon l'une des revendications précédentes **caractérisé en ce que** la donnée physiologique est une mesure de la saturation en oxygène réalisée au moyen d'un capteur utilisant deux rayonnements respectivement rouge et infrarouge.

9. Dispositif selon l'une des revendications précédentes **caractérisé en ce que** la donnée physiologique est une mesure de l'Electro Cardiogramme réalisé au moyen de deux capteurs mis en contact avec les index de l'utilisateur.

10. Dispositif selon l'une des revendications précédentes **caractérisé en ce que** la donnée physiologique est une mesure effectuée sur un prélèvement de larmes et/ou de salives, en vue de produire un examen de mesure de glycémie.

11. Dispositif selon l'une quelconque des revendications précédentes, **caractérisé en ce que** la donnée physiologique stockée dans lesdits moyens de stockage est associé à une information propre à l'utilisateur, par exemple son empreinte digitale capturée par un capteur d'empreinte digitale, de manière à sécuriser les collectes de données physiologiques.

12. Dispositif selon l'une quelconque des revendications précédentes, **caractérisé en ce qu'**il comporte, de fabrication, un défibrillateur intégré.

13. Dispositif selon l'une quelconque des revendications 1 à 11, **caractérisé en ce qu'**il se présente sous la forme d'une tablette tactile dotée de moyen de collecte de données physiologiques, et adaptée pour être disposée au sein d'une housse, d'un étui ou d'une coque comportant un dispositif défibrillateur communiquant avec ladite tablette, et comportant un compartiment scellé de manière à ne permettre qu'un unique accès aux électrodes de défibrillation.

14. Dispositif défibrillateur selon la revendication 13 **caractérisé en ce qu'**il comporte des moyens de détection de l'état de scellement du compartiment d'accès aux électrodes, de manière à commander l'affichage d'un signal avertisseur sur l'écran de ladite tablette.

15. Dispositif selon l'une quelconque des revendications 1 à 11, **caractérisé en ce qu'**il se présente sous la forme d'un ordinateur portable comportant un défibrillateur situé dans un kit batterie ou dans un caddie.
